# EUROPEAN PATENT APPLICATION

(11) **EP 4 813 306 A1**
(43) Date of publication of application: **30.09.2026**
(21) Application number: 26167463.4
(22) Date of filing: 25.03.2026
(51) Int. Cl.: A61B 6/00

(54) **DOSE MANAGEMENT APPARATUS, DOSE SETTING METHOD, AND DOSE SETTING PROGRAM**

(30) Priority: 26.03.2025 JP 2025052224; 31.03.2025 JP 2025058414
(71) Applicant: Konica Minolta, Inc., Tokyo 100-7015 (JP)
(72) Inventor: NAGATSUKA, Sumiya, Tokyo, 100-7015 (JP)
(74) Representative: MFG Patentanwälte Meyer-Wildhagen Meggle-Freund Gerhard PartG mbB

(57) **Abstract**

a dose management apparatus, a dose setting method, and a dose setting program, which are capable of setting an appropriate dose for dose management in dynamic imaging are provided. A dose management apparatus includes: a ratio acquisition section that acquires a ratio between a first reference value of a dose defined for a case where a first site of a subject is imaged as a still image by irradiation with radiation and a second reference value of a dose defined for a case where the first site is imaged as a dynamic image by irradiation with radiation; and a first setting section that sets, based on the ratio and a third reference value of a dose for a case where a second site is imaged as the still image, a fourth reference value of a dose for a case where the second site is imaged as the dynamic image.

## Description

### BACKGROUND OF THE INVENTION

### 1. Field of the Invention

The present invention relates to a dose management apparatus, a dose setting method, and a dose setting program.

### 2. Description of Related Art

A Diagnostic Reference Level (DRL) for optimizing patient's exposure dose is recommended to be used. As the DRL, for example, an exposure dose (DRL value) for a patient of a standard body habitus is defined according to an examination modality, an examination site, and/or the like. A dose management apparatus manages a dose for radiography by comparing an exposure dose of a patient with a DRL value (e.g., Japanese Unexamined Patent Publication No. 2020-044332).

Regarding radiographic imaging, in general imaging for capturing a still image, in order to optimize an exposure dose of a patient, dose management is performed by comparison with the DRL value as described above. Also in dynamic imaging for capturing a dynamic image having a plurality of frame images, it is desired to perform dose management as in the general imaging in order to optimize the exposure dose of a patient. However, the dynamic imaging is a new imaging method which is currently being transitioned from a stage of clinical research to a stage used in a medical site, and a method of dose management, including setting of a DRL value, is not determined.

### SUMMARY OF THE INVENTION

Objectives of the present invention include providing a dose management apparatus, a dose setting method, and a dose setting program, which are capable of setting an appropriate dose for dose management in dynamic imaging.

In order to achieve at least one of the above-described objectives, a dose management apparatus reflecting one aspect of the present invention includes:
a ratio acquisition section that acquires a ratio between a first reference value of a dose defined for a case where a first site of a subject is imaged as a still image by irradiation with radiation and a second reference value of a dose defined for a case where the first site is imaged as a dynamic image by irradiation with radiation; and
a first setting section that sets, based on the ratio and a third reference value of a dose for a case where a second site is imaged as the still image, a fourth reference value of a dose for a case where the second site is imaged as the dynamic image.

In order to achieve at least one of the above-described objectives, a dose setting method reflecting an aspect of the present invention is performed by a dose management apparatus and includes:
obtaining a ratio between a first reference value of a dose defined for a case where a first site of a subject is imaged as a still image by irradiation with radiation and a second reference value of a dose defined for a case where the first site is imaged as a dynamic image by irradiation with radiation, and
setting, based on the ratio and a third reference value of a dose for a case where a second site is imaged as the still image, a fourth reference value of a dose for a case where the second site is imaged as the dynamic image.

In order to achieve at least one of the above-described objectives, a dose setting program reflecting one aspect of the present invention causes a computer of a dose management apparatus to execute:
a process of obtaining a ratio between a first reference value of a dose defined for a case where a first site of a subject is imaged as a still image by irradiation with radiation and a second reference value of a dose defined for a case where the first site is imaged as a dynamic image by irradiation with radiation, and
a process of setting, based on the ratio and a third reference value of a dose for a case where a second site is imaged as the still image, a fourth reference value of a dose for a case where the second site is imaged as the dynamic image.

### BRIEF DESCRIPTION OF DRAWING

The advantages and features provided by one or more embodiments of the invention will become more fully understood from the detailed description given hereinbelow and the appended drawings which are given by way of illustration only, and thus are not intended as a definition of the limits of the present invention:
Fig. 1 is an explanatory diagram illustrating an example of a configuration of a radiographic image processing system according to an embodiment of the present invention;
Fig. 2 is a block diagram illustrating an example of a functional configuration of an imaging controller in a radiographic imaging system constituting the radiographic image processing system;
Fig. 3 is a block diagram illustrating an example of a functional configuration of the radiographic imaging control apparatus constituting the radiographic image processing system;
Fig. 4 is a block diagram illustrating an example of a functional configuration of the radiographic imaging control apparatus constituting the radiographic image processing system;
Fig. 5 is a block diagram illustrating an example (Example 1) of a functional configuration of a dose management apparatus constituting the radiographic image processing system;
Fig. 6 is a diagram illustrating a DRL value (entrance-surface dose) for dynamic imaging set based on a DRL value (entrance-surface dose) for general imaging in the dose management apparatus illustrated in Fig. 5;
Fig. 7 is a view for explaining the DRL value (entrance-surface dose) for dynamic imaging set based on the DRL value (entrance-surface dose) set in a facility for general imaging in the dose management apparatus shown in Fig. 5;
Fig. 8 is a graph displayed for comparison in the dose management apparatus shown in Fig. 5, and is a graph for comparing the DRL values shown in Figs. 6 and 7 with an exposure dose of the patient;
Fig. 9 is a block diagram illustrating another example of the functional configuration of the dose management apparatus constituting the radiographic image processing system (Example 2);
Fig. 10 is a diagram illustrating a DRL value (dose index) set according to each age of a child in head CT;
Fig. 11 is a graph which is displayed for comparison in the dose management apparatus illustrated in Fig. 9 and is a graph for comparing the DRL value illustrated in Fig. 10 and an exposure dose of a patient; and
Fig. 12 is a diagram illustrating a DRL value (entrance-surface dose) in general imaging and a DRL value (dose indicator) set according to an age group of children in chest CT.

### DESCRIPTION OF THE PREFERRED EMBODIMENTS

Hereinafter, one or more embodiments of the present invention will be described with reference to the drawings. However, the scope of the invention is not limited to the disclosed embodiments.

Hereinafter, embodiments of the present invention will be described in detail with reference to the drawings.

### < Configuration of Radiographic Image Processing System >

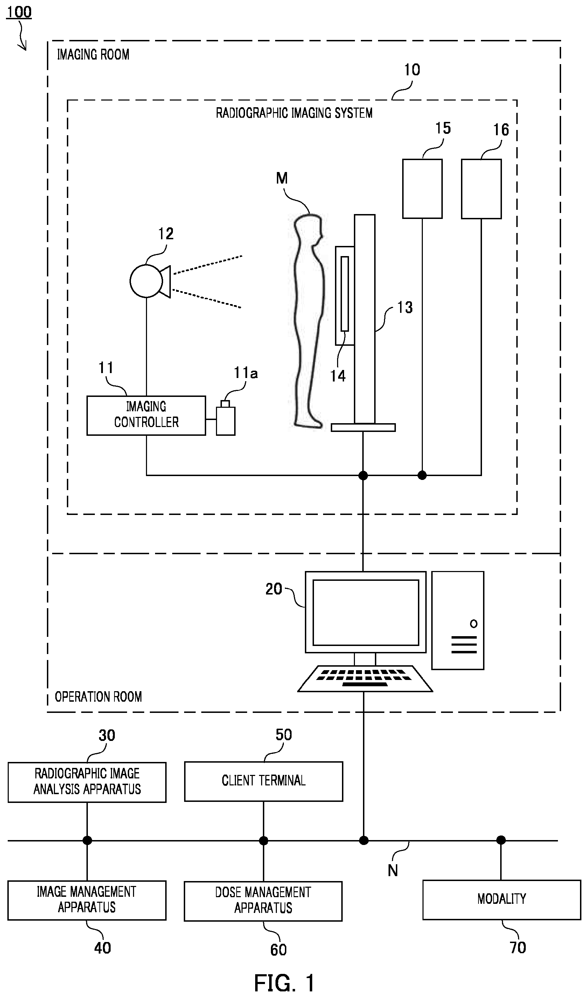

Fig. 1 is a diagram illustrating a radiographic image processing system 100 according to the present embodiment. The radiographic image processing system 100 includes a radiographic imaging system 10, a radiographic imaging control apparatus (console apparatus) 20, a radiographic image analysis apparatus 30, an image management apparatus 40, a client terminal 50, a dose management apparatus 60, and a modality 70.

In the example illustrated in Fig. 1, the radiographic imaging system 10 is arranged in an imaging room, and the radiographic imaging control apparatus 20 is arranged in an operation room. Note that the radiographic imaging system 10 and the radiographic image capturing controller 20 may be provided in a movable cart such as a medical cart.

The radiographic imaging system 10, the radiographic imaging control apparatus 20, the radiographic image analysis apparatus 30, the image management apparatus 40, the client terminal 50, and the dose management apparatus 60 are connected to each other via a communication network N. As the communication network N, for example, a communication network compliant with the Digital Image and Communications in Medicine (DICOM) standard or the like is used.

The modality 70 including a radiographic imaging apparatus other than the radiographic imaging system 10 and the radiographic imaging control apparatus 20 illustrated in Fig. 1 is also connected to the communication network N. The modality 70 includes an X-ray Computed Tomography (CT) apparatus that captures a tomographic image, a general X-ray imaging apparatus that captures a still image, and the like. In the present exemplary embodiment, radiographic imaging that is a target of dose management is dynamic imaging by the radiographic imaging system 10 and the radiographic imaging control device 20 illustrated in Fig. 1, or imaging by the modality 70.

In addition, although not illustrated, a radiation information terminal as a radiation information system that transmits information relating to a radiation examination, for example, examination order information of a patient, to the radiographic image processing system 100 is connected to the communication network N. Examples of the radiology information terminal include a Radiology Information System (RIS).

The radiographic imaging system 10 performs radiographic dynamic imaging (hereinafter referred to as dynamic imaging) which is imaging of a radiographic dynamic image (hereinafter referred to as a dynamic image) on the basis of the control of the radiographic imaging control apparatus 20. The radiographic imaging control apparatus 20 controls the radiographic imaging system 10 based on the examination order information or the like transmitted from the radiation information terminal. The dynamic image generated by the radiographic imaging system 10 is processed by the radiographic imaging control apparatus 20 and transmitted to the radiographic image analysis apparatus 30. The radiographic image analysis device 30 performs dynamic analysis on the dynamic image. The dynamic image and a result of the dynamic analysis are transmitted to and managed by the image management apparatus 40 as a medical image management system. The image management apparatus 40 is, for example, a Picture Archiving and Communication System (PACS). The dynamic image and the result of the dynamic analysis are transmitted to the client terminal 50 and viewed by a healthcare professional such as a physician.

In the present embodiment, dynamic imaging refers to obtaining a moving image composed of a plurality of frame images by repeatedly irradiating a subject with pulsed radiation (for example, X-rays) at a predetermined frame rate (pulse irradiation). Furthermore, the dynamic image refers to a moving image composed of a series of frame images obtained by dynamic imaging. Dynamic analysis described later refers to analysis processing performed on a dynamic image and includes processing of analyzing the dynamic image to emphasize or attenuate (remove) a predetermined structure in addition to processing of analyzing the movement of a subject based on the dynamic image.

Each of the radiographic imaging system 10, the radiographic imaging control apparatus 20, and the radiographic image analysis apparatus 30 is a type of computer that includes a processor and a memory, and realizes a predetermined function by reading, loading, and executing a program stored in the memory.

### [Radiographic Imaging System]

As illustrated in Fig. 1, the radiographic imaging system 10 includes an imaging controller 11, a radiation irradiation section 12, an imaging table 13, a radiation detection section 14, a display section 15, and a sound output section 16.

The imaging controller 11 acquires setting information regarding the setting of the dynamic imaging from the radiographic imaging control apparatus 20. The imaging controller 11 sets imaging conditions for performing dynamic imaging based on the setting information, controls the radiation irradiation section 12 based on the imaging conditions, to irradiate the patient M (subject) with radiation for performing imaging. The imaging controller 11 includes a Central Processing Unit (CPU), a Random Access Memory (RAM), Read Only Memory (ROM), and the like.

The setting information is information on settings for performing dynamic imaging on the patient M. The setting information includes, for example, at least one of a plurality of types of dynamic analyses that can be performed on a dynamic image by the radiographic image analysis apparatus 30. When a plurality of types of dynamic analyses are combined, the setting information may include information on the combination. The setting information is set by an operator of the radiographic image processing system 100, for example, an imaging technician or the like in the radiographic imaging control apparatus 20 to be described later.

The imaging conditions include, for example, conditions related to the patient M such as an imaged site, an imaging direction, a body position of the patient M, a physique of the patient M, a condition of the patient M, an age of the patient M, and a sex of the patient M. The imaged site is, for example, a chest part, a leg part, or the like. The physique of the patient M may be specified by numerical values, such as the weight, height, Body Mass Index (BMI), and body thickness of the patient M, or may be a type according to a range, for example, large, medium, small, thin, average, thick, or the like. The condition of the patient M includes types of breathing, such as breath holding and deep breathing. The age of the patient M may be designated by a numerical value or may be a type in a range of a child, an adult, or the like. The gender of the patient M may be identified from physical structural features.

The imaging conditions also include, for example, conditions related to radiation irradiation such as the type of examination, tube voltage, tube current, imaging time, current-time product, distance, pulse rate, pulse width, pulse interval, the number of imaging frames per imaging, and the dose per unit time in radiation irradiation. Distance is a distance from the irradiation section 12 (radiation source) to the detector 14 (Focus Film Distance: FFD, or Source Image receptor Distance: SID). The pulse rate is the number of times of radiation emission per second, and matches the frame rate of image data. The pulse width is a radiation irradiation duration per radiation irradiation. The pulse interval is a time from the start of one radiation irradiation to the start of the next radiation irradiation, and coincides with a time interval (frame interval) between a plurality of image data.

These imaging conditions may be automatically determined by the imaging controller 11 of the radiographic imaging system 10 based on the setting information.

The radiation irradiation section 12 is disposed at a position facing the radiation detector 14 fixed to the imaging table 13. The irradiation section 12 emits radiation in accordance with an operation of an irradiation instruction switch 11a connected to the imaging controller 11.

To be more specific, based on the operation of the irradiation instruction switch 11a, the irradiation section 12 applies a voltage corresponding to a preset imaging condition to a radiation source (tube), and generates radiation (for example, X-rays) of a dose corresponding to the applied voltage.

The radiation irradiation section 12 generates radiation in a mode corresponding to a radiographic image (a still image, a dynamic image, or the like) to be generated. For example, in the case of a still image, irradiation is performed only once for each depression of the irradiation instruction switch 11a. In the case of a dynamic image, pulsed irradiation is continued after the pressing of the irradiation instruction switch 11a (imaging start instruction) until the release of the pressing (imaging end instruction).

The radiation detection section 14 includes a semiconductor image sensor such as a Flat Panel Detector (FPD), and generates digital data of a radiographic image. The radiation detection section 14 includes a board on which a plurality of detection elements (pixels) are arranged in a matrix, and each pixel of the board detects incident radiation, converts the detected radiation into an electric signal corresponding to the intensity of the detected radiation, and accumulates the electric signal. Each pixel of the board includes, for example, a switching section such as a Thin Film Transistor (TFT).

The radiation detection section 14 controls the switching section of each pixel based on an image reading condition input from the radiographic imaging control apparatus 20 to read the electric signals accumulated in each pixel, and outputs intensity information of each pixel to the image generation section 113. The image reading condition is, for example, a frame rate, a frame interval, a pixel size, an image size (matrix size), or the like. The frame rate is the number of frame images acquired per second, and matches the pulse rate. The frame interval is a time period from the start of one operation of acquiring image data to the start of the operation of acquiring the next frame image, and corresponds to the pulse interval.

The imaging controller 11 and the radiation detection section 14 are connected to each other, and exchange synchronization signals with each other so as to synchronize the radiation irradiation operation and the image reading operation.

As described above, the radiographic imaging system 10 performs dynamic imaging of a radiographic image by the radiation irradiation section 12 emitting radiation and the radiation detection section 14 generating image data based on the detected radiation intensity under the control of the imaging controller 11.

When the dynamic imaging of the patient M is performed, the display section 15 and the sound output section 16 provide the patient M with instructions on a posture to be taken (imaging position), a body state, a breathing state, and the like. The display section 15 is, for example, a display device such as a Cathode Ray Tube (CRT), a liquid crystal display, or an organic Electro Luminescence (EL) display. The sound output section 16 is, for example, a sound output device such as a speaker. The sound output section 16 provides the patient M with an instruction on the physical condition, the respiratory state, or the like by, for example, automatic voice. Each of the display section 15 and the sound output section 16 may provide the instruction having the same content to the patient M, or only one of them may provide the instruction.

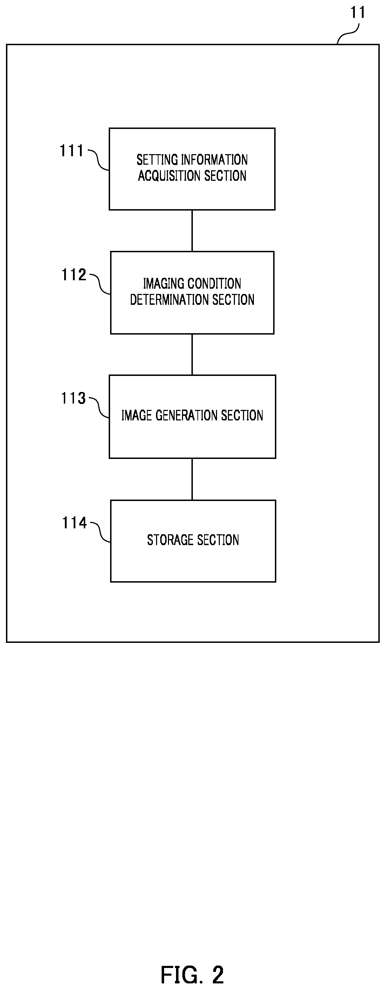

Fig. 2 is a block diagram illustrating an example of a functional configuration of the imaging controller 11 in the radiographic imaging system 10 constituting the radiographic image processing system 100. The imaging controller 11 includes a setting information acquisition section 111, an imaging condition determination section 112, an image generation section 113, and a storage section 114.

The setting information acquisition section 111 acquires setting information from the radiographic imaging control apparatus 20.

The imaging condition determination section 112 determines imaging conditions for performing dynamic imaging of the patient M based on the setting information. Information indicating a correspondence relationship between a plurality of types of dynamic analyses and imaging conditions suitable for the respective dynamic analyses is stored in the storage section 114 in advance. Information indicating a correspondence relationship between combinations of a plurality of types of dynamic analyses and imaging conditions suitable for the combinations is also stored in the storage section 114 in advance. The imaging condition determination section 112 may determine the imaging condition by reading the information indicating the correspondence relationship from the storage section 114 for the dynamic analysis indicated by the setting information or the combination of the plurality of types of dynamic analyses and collating the information with the setting information.

Note that, for example, in the case of screening, emergency, or the like, the dynamic analysis serving as the setting information cannot be set. In such a case, the imaging condition determination section 112 determines the imaging condition by causing the operator to select at least one imaging condition from a plurality of predefined imaging conditions. The imaging condition determination section 112 also allows the operator to select examination order information and determines imaging conditions on the basis of the selected examination order information. As described above, in a case where the dynamic analysis cannot be set before the dynamic imaging, the dynamic analysis is set after the dynamic imaging under the imaging condition selected by the operator, and the dynamic analysis in the radiographic image analysis apparatus 30 is executed.

The image generation section 113 executes dynamic imaging on the patient M on the basis of the determined imaging conditions and generates a plurality of frames of radiographic images. More specifically, the image generation section 113 controls the operations of the radiation irradiation section 12 and the radiation detection section 14 based on the imaging conditions, and generates image data by acquiring, for each pixel, intensity information relating to the intensity of radiation transmitted through the subject from the radiation detection section 14.

As described above, the storage section 114 stores, in advance, information indicating a correspondence relationship between a plurality of types of dynamic analyses and imaging conditions suitable for the respective dynamic analyses, information indicating a correspondence relationship between combinations of the plurality of types of dynamic analyses and imaging conditions suitable for the combinations, and the like.

### [Radiographic Imaging Control Apparatus]

The radiographic imaging control apparatus 20 is, for example, a computer such as a Personal Computer (PC) or a workstation. The radiographic imaging control apparatus 20 may be a desktop computer as in the example illustrated in Fig. 1 or a portable computer such as a laptop personal computer or a tablet computer.

The radiographic imaging control apparatus 20 receives the examination order information from the radiography information terminal and transmits it to the radiographic imaging system 10 to control the dynamic imaging of the radiographic imaging system 10.

The examination order information includes various types of information about dynamic imaging to be performed next, such as instruction information about breathing, patient information, examination information, imaging information, and data attributes. The examination information includes information such as examination IDs, examination target sites (for example, chest, abdomen, lungs, and the heart), and types of analysis (for example, ventilatory analysis, pulmonary blood flow analysis, and maximal voluntary ventilation measurement). The examination order information is generated, for example, when a doctor or the like requests the radiographic image processing system 100 to perform dynamic imaging of the patient M.

In addition, the radiographic imaging control apparatus 20 generates setting information indicating at least one dynamic analysis among a plurality of types of dynamic analyses executable by the radiographic image analysis apparatus 30 on the basis of the input of the operator. In the case of combining a plurality of types of dynamic analyses, the radiographic imaging control apparatus 20 generates setting information indicating the combination of the plurality of types of dynamic analyses. The operator recognizes which dynamic analysis to combine among the plurality of types of dynamic analyses by referring to, for example, the content of the examination order information and performs an input operation for generating the setting information on the basis of the recognition. Alternatively, the operator may recognize which dynamic analyses are to be combined, on the basis of information transmitted from a doctor or the like by another method.

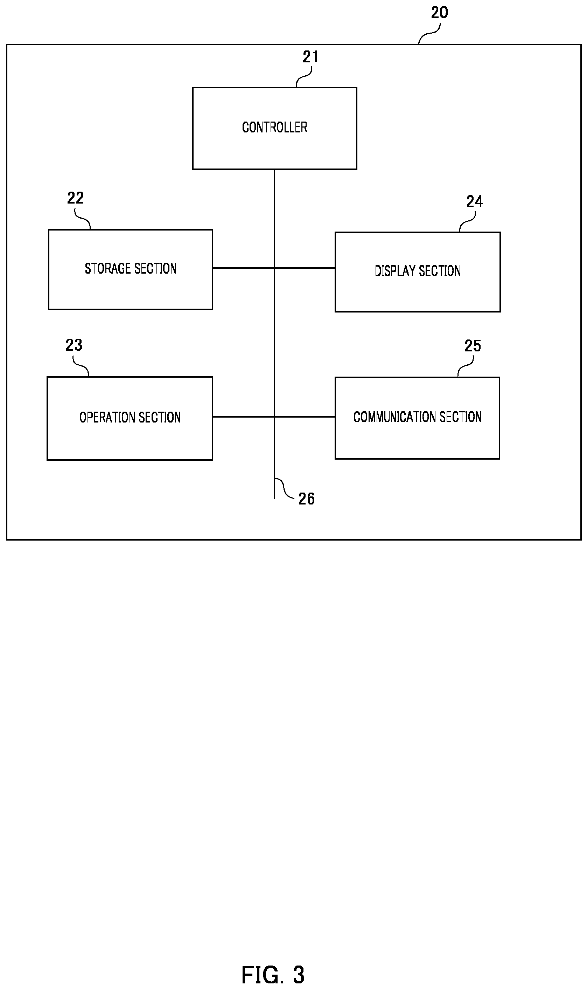

Fig. 3 is a block diagram illustrating an example of a functional configuration of the radiographic imaging control apparatus 20 constituting the radiographic image processing system 100. The radiographic imaging control apparatus 20 includes a controller 21, a storage section 22, an operation section 23, a display section 24, and a communication section 25. The components of the radiographic imaging control apparatus 20 are connected to each other by a bus 26.

The radiographic imaging control apparatus 20 outputs, to the radiographic imaging system 10, the setting conditions set by an operator or the like and examination order information acquired in advance from the radiation information terminal, and controls imaging processing by the radiographic imaging system 10. The radiographic imaging control apparatus 20 may display the dynamic image generated by the radiographic imaging system 10, for example, for the operator to confirm it.

The controller 21 includes a CPU, a RAM, and the like. In the controller 21, in response to an operation of the operation section 23, the CPU reads a system program and various processing programs stored in the storage section 22, loads them in the RAM, and controls the operation of each section of the radiographic imaging control apparatus 20 based on the loaded program.

The storage section 22 is configured by a nonvolatile semiconductor memory, a hard disk, or the like. The storage section 22 stores various programs to be executed by the controller 21, parameters required for execution of processing by the programs, or data such as processing results (dynamic images and the like). The various programs are stored in the form of readable program codes, and the controller 21 sequentially executes operations in accordance with the program codes.

Further, the storage section 22 stores image reading conditions for performing dynamic imaging. Furthermore, the storage section 22 stores the examination order information transmitted from the radiation information terminal. When the radiographic imaging control apparatus 20 controls the dynamic imaging of the radiographic imaging system 10, the radiographic imaging control apparatus 20 reads the image reading condition and the examination order information corresponding to the patient M from the storage section 22 and transmits the read information.

The operation section 23 is an operation device such as a keyboard including cursor keys, number input keys, and various function keys, a pointing device such as a mouse or a trackball, and a touch screen. The operation section 23 generates an instruction signal based on an input of an operator, and outputs the instruction signal to the controller 21.

The display section 24 is constituted by a display device such as a CRT, a liquid crystal display, or an organic EL display. The display section 24 displays an input instruction from the operation section 23, image data (a dynamic image or the like) generated by the radiographic imaging system 10, or the like according to an instruction of a display signal input from the controller 21.

The communication section 25 transmits and receives data to and from the radiographic imaging system 10, the radiographic image analysis apparatus 30, and the like.

### [Radiographic Image Analysis Apparatus]

The radiographic image analysis apparatus 30 is, for example, a computer such as a PC or a workstation. The radiographic image analysis apparatus 30 may be a desktop computer or a portable computer such as a laptop personal computer or a tablet computer.

The radiographic image analysis apparatus 30 performs dynamic analysis on the dynamic image captured by the radiographic imaging system 10 based on the setting information set in the radiographic imaging control apparatus 20.

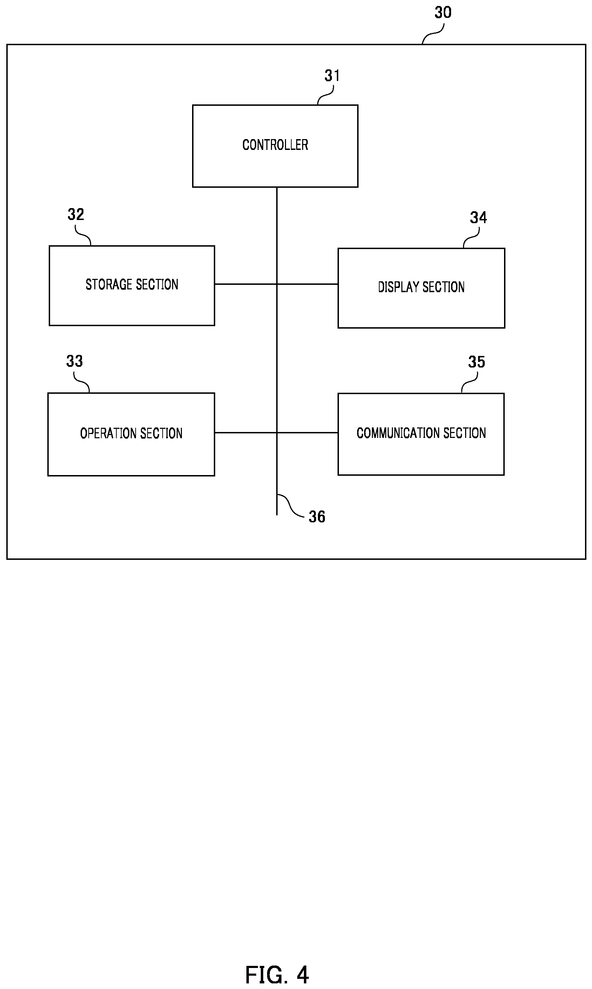

Fig. 4 is a block diagram illustrating an example of a functional configuration of the radiographic image analysis apparatus 30 constituting the radiographic image processing system 100. The radiographic image analysis apparatus 30 includes a controller 31, a storage section 32, an operation section 33, a display section 34, and a communication section 35. These components of the radiographic image analysis apparatus 30 are connected to each other by a bus 36.

The controller 31 includes a CPU, a RAM, and the like. In the controller 31, in response to an operation of the operation section 33, the CPU reads a system program and various processing programs stored in the storage section 32, loads them in the RAM, and executes operation control of each part of the radiographic image analysis apparatus 30, dynamic analysis, and the like on the basis of the loaded programs.

The controller 31 acquires dynamic images that are a plurality of frames of radiographic images generated by the radiographic imaging system 10 and the radiographic imaging controller 20, executes the dynamic analysis set in the setting information on the dynamic images, and acquires an analysis result. The controller 31 performs analysis, for example, on the basis of signal changes in a plurality of frame images.

The controller 31 has, for example, a blood flow analysis mode, a ventilation analysis mode, an adhesion analysis mode, a diaphragm motion amount analysis mode, a shaping-related measurement mode, and the like as the type of dynamic analysis. Each mode will be briefly described below.

The blood flow analysis mode is a mode in which a signal change in the lung field synchronized with the heartbeat is visualized.

The ventilation analysis mode is a mode in which a signal change in a time direction in a specific timefrequency band is extracted and lung tissue behavior during breathing is visualized.

The adhesion analysis mode is a mode for visualizing the degree of adhesion of tissues.

The diaphragm motion amount analysis mode is a mode for tracking the up/down motion of the diaphragm associated with breathing.

The shaping-related measurement mode is, for example, a mode in which a change in the position of a specified bone in the four limbs or the like is measured and the trajectory of the movement is displayed.

The storage section 32 is configured by a nonvolatile semiconductor memory, a hard disk, or the like. The storage section 32 stores various programs executed by the controller 31, parameters necessary for execution of processing by the programs, or data such as processing results (dynamic images, analysis results, and the like). The various programs are stored in the form of readable program codes, and the controller 31 sequentially executes operations in accordance with the program codes.

In addition, the storage section 32 stores patient information or examination information related to each dynamic image generated by the radiographic imaging system 10, and list information indicating a status (for example, a progress state such as during reception, during dynamic analysis, or analysis completion).

The operation section 33 is an operation device such as a keyboard including cursor keys, number input keys, and various function keys, a pointing device such as a mouse or a trackball, and a touch screen. The operation section 33 generates an instruction signal based on an input of an operator, and outputs the instruction signal to the controller 31. Furthermore, the operation section 33 may include a touch screen on the display screen of the display section 34, and in this case, outputs the instruction signal input through the touch screen to the controller 31.

The display section 34 is constituted by a display device such as a CRT, a liquid crystal display, or an organic EL display. The display section 34 displays an input instruction from the operation section 33, data (a dynamic image, an analysis result, and the like) generated by the radiographic imaging system 10, and the like in accordance with an instruction of a display signal input from the controller 31.

The communication section 35 transmits and receives data to and from the radiographic imaging control apparatus 20, the image management apparatus 40, and the like.

### [Image Management Apparatus and Client Terminal]

The image management apparatus 40 and the client terminal 50 are, for example, computers such as a PC and a workstation. Each of the image management apparatus 40 and the client terminal 50 may be a desktop computer or a portable computer such as a laptop personal computer or a tablet computer. The image management apparatus 40 and the client terminal 50 may be a known image management apparatus and client terminal, respectively, and thus detailed description thereof is omitted herein.

### < Example 1 >

### [Dose Management Apparatus]

In the present embodiment, the dose management apparatus 60 is, for example, a computer such as a PC or a workstation. The dose management apparatus 60 may be a desktop computer or a portable computer such as a laptop personal computer or a tablet computer.

The dose management apparatus 60 records and manages dose information of a radiation dose associated with imaging of a radiographic image such as a still image or a dynamic image. Specifically, the dose management apparatus 60 has a recording function of recording dose information and a management function of managing dose information. The recording function records and accumulates information including dose information associated with the capturing of the radiographic image for an individual patient. The management function manages the dose information by comparing the accumulated dose information of the individual patient with the set diagnostic reference level.

Here, for example, a diagnostic reference level corresponding to an examination site is defined for general imaging, but a diagnostic reference level is not defined for dynamic imaging for capturing a dynamic image, and thus it is difficult to manage a dose.

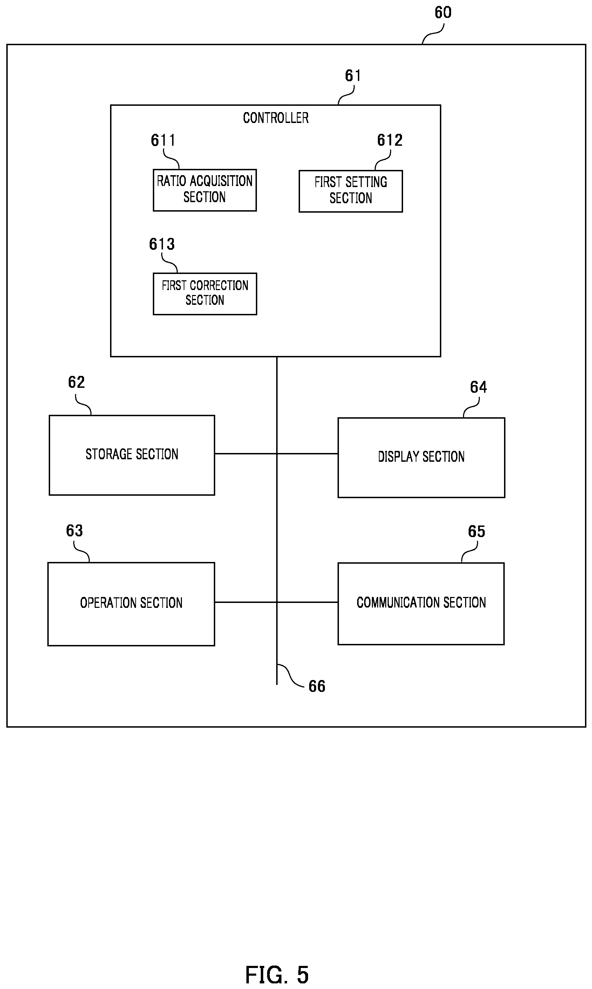

Therefore, in the present embodiment, the dose management apparatus 60 further includes a setting function (the ratio acquisition section 611, the first setting section 612, and the first correction section 613) of setting the diagnostic reference level for the dynamic imaging apparatus in which the diagnostic reference level is not set. The setting function (the ratio acquisition section 611, the first setting section 612, and the first correction section 613) will be described below with reference to Fig. 5.

Fig. 5 is a block diagram illustrating an example (Example 1) of a functional configuration of the dose management apparatus 60 constituting the radiographic image processing system 100. The dose management apparatus 60 includes a controller 61, a storage section 62, an operation section 63, a display section 64, and a communication section 65. These components of the dose management apparatus 60 are connected to each other by a bus 66.

The controller 61 is constituted by a CPU, a RAM, and the like. In the controller 61, in response to an operation of the operation section 63, the CPU reads a system program and various processing programs stored in the storage section 62, loads them in the RAM, and executes operation control of each part of the dose management apparatus 60, dose management, and the like based on the loaded programs.

The controller 61 includes a ratio acquisition section 611, a first setting section 612, and a first correction section 613. Note that the first diagnostic reference level L1 to the fifth diagnostic reference level L5 below are examples of the first reference value to the fifth reference value, respectively.

The ratio acquisition section 611 reads, for example, the first diagnostic reference level L1 and the second diagnostic reference level L2 stored in the storage section 62. The first diagnostic reference level L1 is a diagnostic reference level of a dose defined for a case where a predetermined site (an example of a first site) of a subject is imaged as a still image by irradiation with radiation. The second diagnostic reference level L2 is a diagnostic reference level of a dose defined for a case where a predetermined site is imaged as a dynamic image by irradiation with radiation. Then, the ratio acquisition section 611 obtains the ratio R = L2/L1.

Further, the ratio acquisition section 611 may obtain the ratio R = L2/L5 using the fifth diagnostic reference level L5 instead of the first diagnostic reference level L1. The fifth diagnostic reference level L5 is a diagnostic reference level of a dose defined for a case where a predetermined site of a subject is imaged as a still image by irradiation with radiation, and is a diagnostic reference level defined uniquely by an imaging facility.

For example, the first setting section 612 reads the third diagnostic reference level L3 stored in the storage section 62. The third diagnostic reference level L3 is a diagnostic reference level of a dose for a case where a target site (an example of a second site) different from the predetermined site is imaged as a still image. Then, based on the third diagnostic reference level L3 and the ratio R, the first setting section 612 sets the fourth diagnostic reference level L4 of a dose to be used for dose management in the case of imaging the target site as the dynamic image. Specifically, the fourth diagnostic reference level L4 = L3 × R. Here, the target site is a site different from the predetermined site, but may be the same site as the predetermined site.

The first correction section 613 corrects the fourth diagnostic reference level L4 according to the target site (for example, the body thickness of the target site). When the target site is the chest, the first correction section 613 may correct the fourth diagnostic reference level L4 according to the respiratory state of the subject.

The storage section 62 is configured by a nonvolatile semiconductor memory, a hard disk, or the like. The storage section 62 stores various programs (for example, a dose setting program for executing a dose setting method) executed by the controller 61, parameters necessary for execution of processing by the programs, and the like. The various programs are stored in the form of readable program codes, and the controller 61 sequentially executes operations in accordance with the program codes.

In addition, the storage section 62 stores dose information of a radiation dose at the time in which a still image or a dynamic image received from the radiographic imaging control apparatus 20 is captured. The storage section 62 stores, for example, a still image or a dynamic image received from the radiographic imaging control apparatus 20 and the corresponding dose information in association with each other. Note that the storage section 62 may store dose information of the radiation dose received by the patient in a case other than capturing of a still image or a dynamic image.

The storage section 62 also stores the first diagnostic reference level L1 to the fifth diagnostic reference level L5, the ratio R, and the like. The first diagnostic reference level L1 is, for example, a diagnostic reference level (DRL value) for each examination item of general imaging defined by the DRLs 2020 (e.g., items of conditions such as imaged site and age of patient to be imaged).

The operation section 63 is an operation device such as a keyboard including cursor keys, number input keys, and various function keys, a pointing device such as a mouse or a trackball, and a touch screen. The operation section 63 generates an instruction signal based on an input of an operator, and outputs the instruction signal to the controller 61. Furthermore, the operation section 63 may include a touch screen on the display screen of the display section 64, and in this case, outputs the instruction signal input through the touch screen to the controller 61.

The display section 64 is constituted by a display device such as a CRT, a liquid crystal display, or an organic EL display. The display section 64 displays an input instruction from the operation section 63, data held by the dose management apparatus 60, and the like in accordance with an instruction of a display signal input from the controller 61.

The communication section 65 transmits and receives data to and from the radiographic imaging control apparatus 20, the image management apparatus 40, and the like.

In the example illustrated in Fig. 1, the dose management apparatus 60 is an independent apparatus different from the radiographic imaging control apparatus 20 and the image management apparatus 40, but may not be an independent apparatus and may be included in the radiographic imaging control apparatus 20, the image management apparatus 40, or the like.

### [Dose Setting Method in Example 1]

### (Setting Example 1)

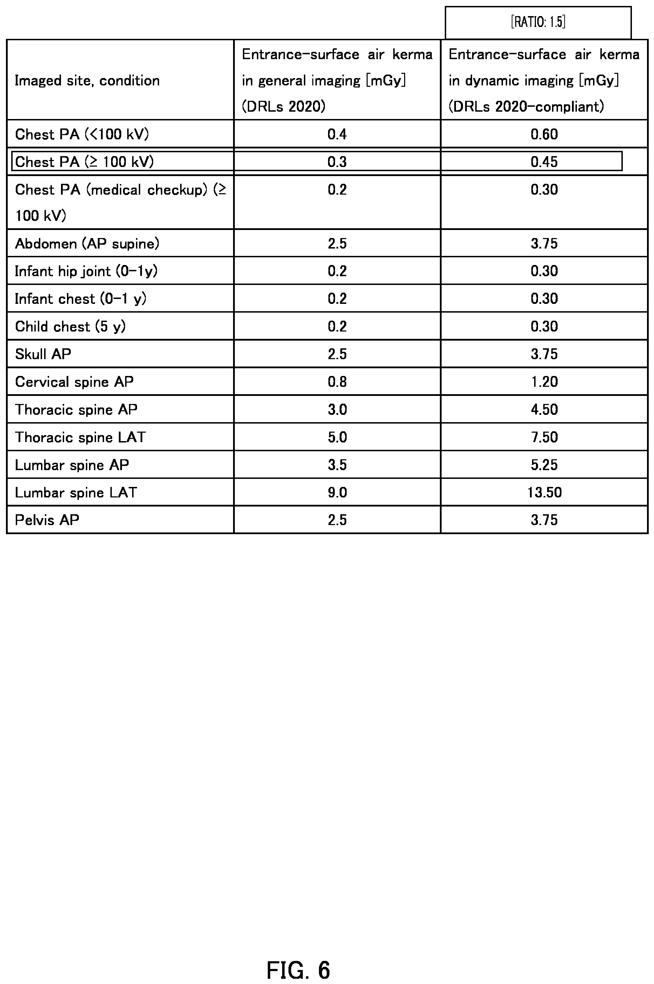

A setting method for setting a diagnostic reference level for dynamic imaging for which no diagnostic reference level is set in the dose management apparatus 60 will be described with reference to Fig. 6. Fig. 6 is a diagram illustrating a DRL value (entrance-surface dose, which is also expressed as "entrance-surface air kerma" in the figures, the terms being used interchangeably) for dynamic imaging set based on a DRL value (entrance-surface dose) for general imaging in the dose management apparatus 60 illustrated in Fig. 5. Note that here, since the entrance-surface dose is used as the DRL value, the entrance-surface dose will be described as an example, but the DRL value may be any other dose, and may, for example, be the Dose-Area Product (DAP).

As illustrated in Fig. 6, for the general imaging, the entrance-surface doses described in the respective columns of "Chest PA (< 100 kV)" to "Pelvis PA" are defined as the DRL values.

In general imaging, "0.3 mGy" is defined as the DRL value (first diagnostic reference level L1) as the entrance-surface dose for "Chest PA (≥ 100 kV)". In addition, it is assumed that "0.45 mGy" is set as the DRL value (the second diagnostic reference level L2) as the entrance-surface dose of "Chest PA (≥ 100 kV)" in the dynamic imaging in the radiographic imaging system 10 and the radiographic imaging control apparatus 20 included in a certain facility. In this case, the ratio acquisition section 611 of the controller 61 obtains the ratio R = L2/L1. In this example, the ratio R = 1.5.

Then, the first setting section 612 of the controller 61 sets the DRL value (fourth diagnostic reference level L4) of each field in the dynamic imaging by multiplying the DRL value (third diagnostic reference level L3) of each field defined for the general imaging by the ratio R using the ratio R. For example, since the DRL value for the general imaging is "0.4 mGy" under the condition of "Chest PA (less than 100 kV)", the DRL value for the dynamic imaging becomes "0.6 mGy" by multiplying the ratio R = 1.5.

As described above, the controller 61 sets the fourth diagnostic reference level L4 serving as the diagnostic reference level for the dynamic imaging in which the diagnostic reference level is not set.

As described above, in the present embodiment, the dose management apparatus 60 includes the ratio acquisition section 611 and the first setting section 612. Here, the first diagnostic reference level L1 of a dose set for a case where a predetermined site of the subject is imaged as a still image by irradiation with radiation (general imaging) and the second diagnostic reference level L2 of a dose set for a case where a predetermined site of the subject is imaged as a dynamic image by irradiation with radiation (dynamic imaging) are set. The ratio acquisition section 611 obtains the ratio R = L2/L1 between the first diagnostic reference level L2 and the second diagnostic reference level L1. The first setting section 612 sets the fourth diagnostic reference level L4 of the dose for the case of imaging the target site as the dynamic image, based on the third diagnostic reference level L3 of the dose for the case of imaging the target site as the still image (general imaging) and the ratio R set by the ratio acquisition section. To be specific, the first setting section 612 multiplies the third diagnostic reference level L3 by the ratio R to set the fourth diagnostic reference level L4 of the dose to be used for the radiation dose management in the case of imaging the target site as the dynamic image.

Since the dose management apparatus 60 has the above-described configuration, it is possible to set an appropriate diagnostic reference level for dose management of dynamic imaging.

### (Setting Example 2)

In the example illustrated in Fig. 6, the defined DRL value (first diagnostic reference level L1) is used as it is for general imaging. However, depending on the imaging facility, the DRL value (the first diagnostic reference level L1) defined for general imaging may not be used as it is, and the DRL value (the fifth diagnostic reference level L5) unique to the imaging facility may be used.

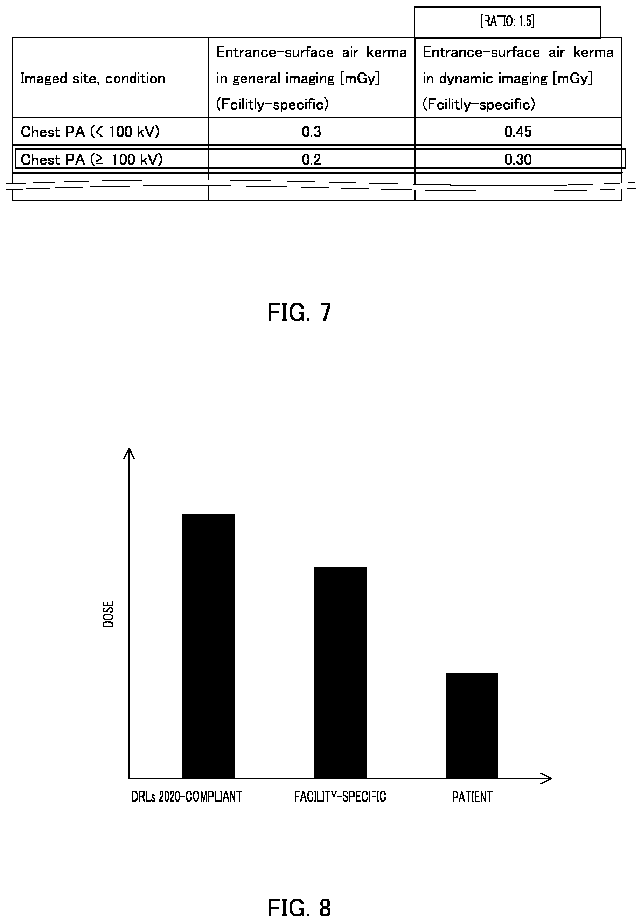

In this case, when the diagnostic reference level is set for the dynamic imaging in which the diagnostic reference level is not set, the DRL value (the fifth diagnostic reference level L5) unique to the imaging facility is used instead of the DRL value (the first diagnostic reference level L1) defined for the general imaging. A setting method for setting a diagnostic reference level for dynamic imaging for which no diagnostic reference level is set in this case will be described with reference to Fig. 7. Fig. 7 is a view for explaining the DRL value (entrance-surface dose) for dynamic imaging set based on the DRL value (entrance-surface dose) set in a facility for general imaging in the dose management apparatus 60 shown in Fig. 5.

The present setting example is basically the same as the above-described setting example 1. Here, in general imaging, as the entrance-surface dose in "Chest PA (≥ 100 kV)", "0.2 mGy" that is unique to an imaging facility is defined as the DRL value (fifth diagnostic reference level L5). In addition, it is assumed that "0.30 mGy" is set as the DRL value (second diagnostic reference level L2) as the entrance-surface dose of "Chest PA (≥ 100 kV)" in the dynamic imaging in the radiographic imaging system 10 and the radiographic imaging control apparatus 20 included in the facility. In this case, the ratio acquisition section 611 of the controller 61 obtains the ratio R = L2/L5. In this example, the ratio R = 1.5.

Then, the first setting section 612 of the controller 61 sets the DRL value (fourth diagnostic reference level L4) of each field in the dynamic imaging by multiplying the DRL value (fifth diagnostic reference level L5) of each field set for the general imaging by the ratio R = 1.5 using the ratio R = 1.5. For example, since the DRL value of the general imaging is "0.3 mGy" in "Chest PA (less than 100 kV)", the DRL value of the radiographic imaging apparatus becomes "0.45 mGy" by multiplying the ratio R = 1.5.

As described above, the controller 61 sets the fourth diagnostic reference level L4 serving as the diagnostic reference level for the dynamic imaging in which the diagnostic reference level is not set.

Next, the dose management apparatus 60 manages the dose information of the patient using the diagnostic reference levels set as described above.

Specifically, when the radiographic imaging system 10 and the radiographic imaging control apparatus 20 capture a still image or a dynamic image, the controller 21 of the radiographic imaging control apparatus 20 calculates an entrance-surface dose at the time of imaging on the basis of an imaging execution condition which is an actual imaging condition at the time of imaging. The controller 21 calculates the entrance-surface dose at the time of imaging on the basis of, for example, the type of examination, the FFD (or the SID), the tube voltage, the current-time product, the frame rate, the imaging time, the patient's position, the patient's physique, the patient's condition, the patient's age, the patient's sex, and the like which are the imaging execution conditions.

The controller 21 of the radiographic imaging control apparatus 20 transmits the imaging execution conditions, the entrance-surface dose, and the like to the radiographic image analysis apparatus 30, the image management apparatus 40, the dose management apparatus 60, and the like in association with the captured still image or dynamic image via the communication section 25. Furthermore, in response to the operation, the dose management apparatus 60 may acquire the imaging execution conditions, the entrance-surface dose, and the like together with the still image and the dynamic image from the radiographic imaging control apparatus 20, the image management apparatus 40, or the like.

The controller 61 of the dose management apparatus 60 stores and accumulates the transmitted or acquired still image, dynamic image, imaging execution conditions, entrance-surface dose, and the like in the storage section 62, and displays information for comparing the set diagnostic reference level with the entrance-surface dose on the display section 24 for the patient who is the dose management target.

Here, Fig. 8 is a graph displayed for comparison in the dose management apparatus 60 shown in Fig. 5, and is a graph for comparing the DRL value shown in Figs. 6 and 7 with the exposure dose of the patient.

In this manner, the controller 61 provides information on dose management to the operator of the dose management apparatus 60 by displaying, on the display section 24, a graph for comparing the set diagnostic reference level with the exposure dose for a patient who is a dose management target. For example, the controller 61 may provide the operator of the dose management apparatus 60 with information for reviewing the currently set diagnostic reference level by displaying the exposure dose of a plurality of patients in a histogram for each examination item. Furthermore, based on the set diagnostic reference level and the exposure dose of the patient, the controller 61 may calculate the number of still images and dynamic images (the number of frames) and an imaging time that are allowable thereafter, and provide the calculation results to the radiographic imaging control apparatus 20.

### (Setting Example 3)

In the setting example 1 and the setting example 2, the influence of each site is not considered, but the diagnostic reference level may be set in consideration of the influence of each site.

For example, since the amount of radiation transmitted through the chest varies depending on the respiratory state, it is better to correct the diagnostic reference level according to the respiratory state. Specifically, since the amount of transmitted radiation is larger in imaging in the state of inhaling than in imaging in the state of exhaling, the diagnostic reference level can be set lower. For example, in X-ray imaging of the posteroanterior view of the chest in a medical checkup, imaging is performed in a state of inhaling, and therefore the diagnostic reference level can be set lower.

In this setting example, the controller 61 includes a first correction section 613, and the first correction section 613 corrects the DRL value (fourth diagnostic reference level L4) using a correction factor α for imaging of the posteroanterior view of the chest in a state of inhaling. The correction factor α in this case is, for example, 0.9. In this case, since the DRL value (the fourth diagnostic reference level L4) for the dynamic imaging is "0.30" for "Chest PA (medical checkup) (≥ 100 kV)" illustrated in Fig. 6, this is multiplied by the correction factor α= 0.9, and "0.27" is set as the DRL value after correction.

Furthermore, since the transmission amount of X-rays also changes depending on the body thickness, the DRL value (the fourth diagnostic reference level L4) may be corrected according to the body thickness. For example, since the lateral view of the lumbar spine has a thicker body thickness than other portions, the first correction section 613 of the controller 61 corrects the DRL value (fourth diagnostic reference level L4) using the correction factor α also for imaging of the lateral view of the lumbar spine. When the correction factor α in this case is, for example, 1.3, the DRL value (fourth diagnostic reference level L4) for the dynamic imaging is "13.5" for the "Lumbar spine LAT" shown in Fig. 6, and "17.55" is thus set as the corrected DRL value by multiplying the DRL value by the correction factor α= 1.3.

As described above, the controller 61 corrects the fourth diagnostic reference level L4 serving as the diagnostic reference level in accordance with the site.

### (Setting Example 4)

In the setting example 1, the target site (an example of the second site) is a site different from the predetermined site (an example of the first site), but the target site may be the same site as the predetermined site.

Also in the present setting example, the ratio acquisition section 611 obtains the ratio R = L2/L1 on the basis of the first diagnostic reference level L2 and the second diagnostic reference level L1, similarly to the above-described setting example 1.

Also in this setting example, the first setting section 612 reads, for example, the third diagnostic reference level L3 stored in the storage section 62. However, the third diagnostic reference level L3 is a value uniquely set in the imaging facility in the case of imaging the target site, which is the same site as the predetermined site, as a still image (general imaging), and is a value different from the first diagnostic reference level L1. Then, based on the third diagnostic reference level L3 and the ratio R, the first setting section 612 sets the fourth diagnostic reference level L4 (= L3 × R) of the dose to be used for the dose management in the case of imaging the target site as the dynamic image.

For example, in general imaging, "0.3 mGy" is defined as the DRL value (first diagnostic reference level L1) as the entrance-surface dose of "Chest PA (≥ 100 kV)". In the dynamic imaging in the radiographic imaging system 10 and the radiographic imaging control apparatus 20, "0.45 mGy" is defined as the DRL value (second diagnostic reference level L2) as the entrance-surface dose of "Chest PA (≥ 100 kV)". In this case, the ratio acquisition section 611 of the controller 61 obtains the ratio R = L2/L1. In this example, the ratio R = 1.5.

The first setting section 612 of the controller 61 sets the DRL value (the fourth diagnostic reference level L4) for the dynamic imaging by multiplying the DRL value (the third diagnostic reference level L3) set for the general imaging by the ratio R with respect to "Chest PA (≥ 100 kV)". For example, it is assumed that the DRL value (the third diagnostic reference level L3) set for general imaging for "Chest PA (≥ 100 kV)" is "0.2 mGy". The first setting section 612 multiplies "0.2 mGy" by the ratio R = 1.5 to obtain and set "0.3 mGy" as the DRL value (fourth diagnostic reference level L4) of the dynamic imaging for "Chest PA (≥ 100 kV)".

As described above, the controller 61 also assigns the fourth diagnostic reference level L4, which is the diagnostic reference level unique to the imaging facility, to dynamic imaging for which the diagnostic reference level is defined.

In the above description, the fourth diagnostic reference level L4 is set as a dose value for dose management, but may be set as a dose value serving as an imaging condition for dynamic imaging.

### < Example 2 >

### [Dose Management Apparatus]

Also in the present embodiment, the dose management apparatus 60 may be, for example, a computer such as a PC or a workstation. The dose management apparatus 60 may be a desktop computer or a portable computer such as a laptop personal computer or a tablet computer. That is, the dose management apparatus 60 in the present example may have a configuration equivalent to that of the dose management apparatus 60 described in Example 1, and therefore, a configuration equivalent to that of the dose management apparatus 60 described in Example 1 is denoted by the same reference sign and described below.

As described in Example 1, the dose management apparatus 60 has a recording function of recording dose information and a management function of managing the dose information, and records and manages the dose information on a radiation dose associated with imaging of a radiographic image.

Regarding radiographic imaging, in general imaging for capturing a still image, in order to optimize an exposure dose of a patient, dose management is performed by comparison with the DRL value as described in the background art. However, in general imaging, a DRL value (diagnostic reference level) is not defined depending on an examination item (for example, an item of a condition such as an imaged site or an age of a patient to be imaged), and dose management of radiographic image imaging is not easy. In addition, the same applies to other radiographic imaging, for example, an X-ray CT apparatus or the like, and a DRL value (diagnostic reference level) is not defined depending on an examination item, and dose management of radiographic imaging is not easy.

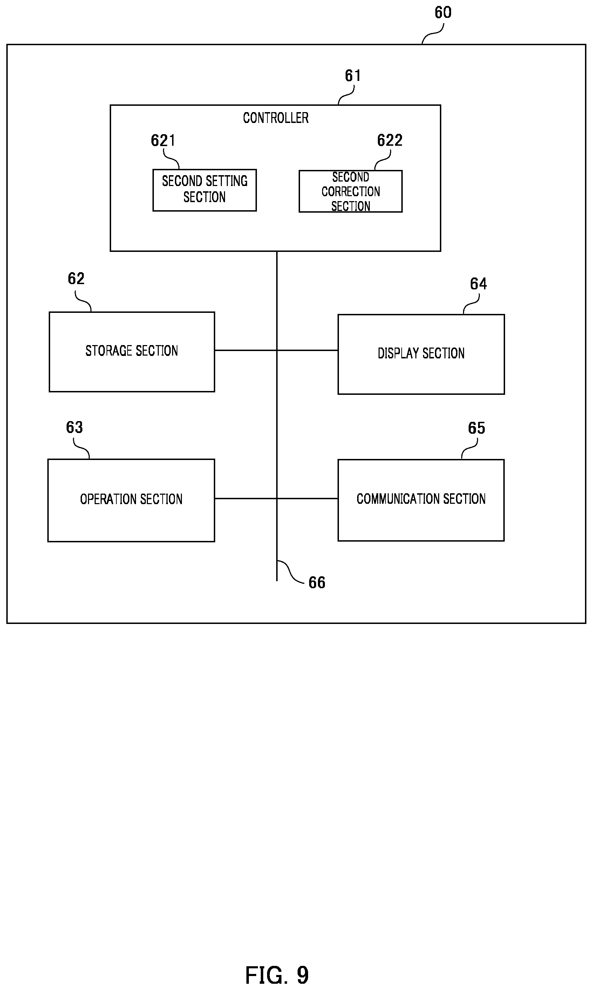

Therefore, in the present embodiment, the dose management apparatus 60 further includes a setting function (the second setting section 621 and the second correction section 622) of setting a diagnostic reference level for an examination item for which a diagnostic reference level has not been set. The setting function (the second setting section 621 and the second correction section 622) will be described below with reference to Fig. 9.

Fig. 9 is a block diagram illustrating another example (Example 2) of the functional configuration of the dose management apparatus 60 constituting the radiographic image processing system 100. The dose management apparatus 60 includes, as in Example 1, the controller 61, the storage section 62, the operation section 63, the display section 64, and the communication section 65. These components of the dose management apparatus 60 are connected to each other by a bus 66.

The controller 61 is constituted by a CPU, a RAM, and the like. In the controller 61, in response to an operation of the operation section 63, the CPU reads a system program and various processing programs stored in the storage section 62, loads them in the RAM, and executes operation control of each part of the dose management apparatus 60, dose management, and the like based on the loaded programs.

The controller 61 includes a second setting section 621 and a second correction section 622. Note that the sixth diagnostic reference level L6 to the eighth diagnostic reference level L8 below are examples of a sixth reference value to an eighth reference value, respectively.

The second setting section 621 calculates a seventh diagnostic reference level L7 of the dose for the age of the subject on the basis of the sixth diagnostic reference level L6 of the dose defined for each age group for a case where the target site of the subject is imaged by irradiation with radiation in the radiographic imaging apparatus and the age of the subject. To be specific, the second setting section 621 calculates the seventh diagnostic reference level L7 using the sixth diagnostic reference level L6 of the dose for the age group as a value corresponding to the median value, the smallest value, or the largest value of the age group. Then, the second setting section 621 sets the calculated seventh diagnostic reference level L7 as a value used for the radiation dose management when the target site is imaged by the radiographic imaging apparatus.

The second setting section 621 may calculate the seventh diagnostic reference level L7 by using the eighth diagnostic reference level L8 instead of the sixth diagnostic reference level L6. The eighth diagnostic reference level L8 is a diagnostic reference level of the dose defined for each age group in a case where the target site is imaged by a radiographic imaging apparatus different from the above-described radiographic imaging apparatus.

The second correction section 622 corrects the seventh diagnostic reference level L7 according to the target site (for example, the body thickness of the target site). When the target site is the chest, the second correction section 622 may correct the seventh diagnostic reference level L7 according to the respiratory state of the subject.

The storage section 62 is configured by a nonvolatile semiconductor memory, a hard disk, or the like. The storage section 62 stores various programs (for example, a dose setting program for executing a dose setting method) executed by the controller 61, parameters necessary for execution of processing by the programs, and the like. The various programs are stored in the form of readable program codes, and the controller 61 sequentially executes operations in accordance with the program codes.

Further, the storage section 62 stores dose information of a radiation dose at the time of imaging in a radiographic image received from the radiographic imaging control apparatus 20 or the modality 70. The storage section 62 stores, for example, a radiographic image received from the radiographic imaging control apparatus 20 or the modality 70 and corresponding dose information in association with each other. Note that the storage section 62 may store dose information of the radiation dose received by the patient other than the radiographic image capturing.

The storage section 62 also stores the sixth diagnostic reference level L6 to the eighth diagnostic reference level L8, and the like. The sixth diagnostic reference level L6 and the eighth diagnostic reference level L8 are, for example, diagnostic reference levels (DRL values) defined in DRLs 2020.

The operation section 63 is an operation device such as a keyboard including cursor keys, number input keys, and various function keys, a pointing device such as a mouse or a trackball, and a touch screen. The operation section 63 generates an instruction signal based on an input of an operator, and outputs the instruction signal to the controller 61. Furthermore, the operation section 63 may include a touch screen on the display screen of the display section 64, and in this case, outputs the instruction signal input through the touch screen to the controller 61.

The display section 64 is constituted by a display device such as a CRT, a liquid crystal display, or an organic EL display. The display section 64 displays an input instruction from the operation section 63, data held by the dose management apparatus 60, and the like in accordance with an instruction of a display signal input from the controller 61.

The communication section 65 transmits and receives data to and from the radiographic imaging control apparatus 20, the image management apparatus 40, the modality 70, and the like.

In the example illustrated in Fig. 1, the dose management apparatus 60 is an independent apparatus different from the radiographic imaging control apparatus 20, the image management apparatus 40, and the modality 70. However, the dose management apparatus 60 may not be an independent apparatus, and may be included in the radiographic imaging control apparatus 20, the image management apparatus 40, the modality 70, or the like.

### [Dose Setting Method in Example 2]

### (Setting Example 5)

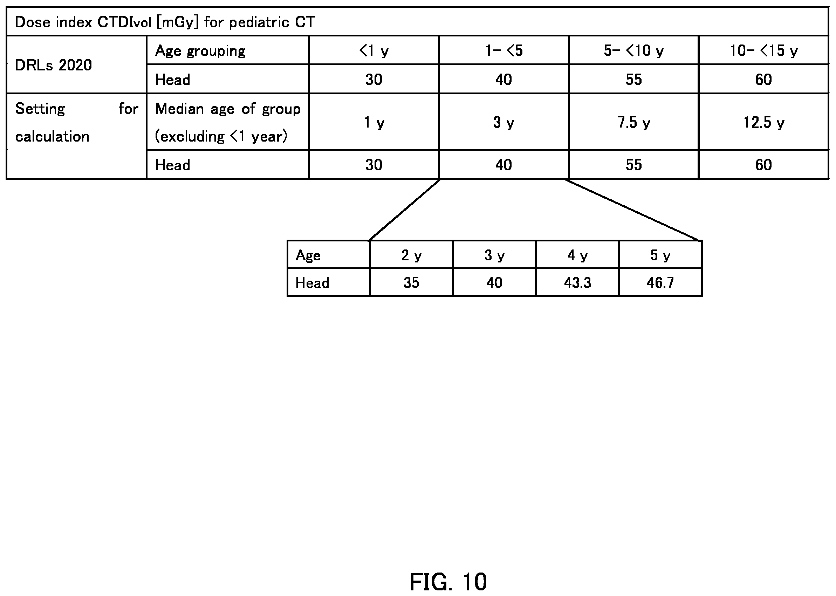

Fig. 10 is a diagram illustrating DRL values set according to the ages of children for head CT. In Fig. 10, Computed Tomography Dose Index volume (CTDIvol) used as the DRL value is one of the indices of the exposure dose in CT.

Among radiographic imaging apparatuses that capture radiographic images, for example, there are radiographic imaging apparatuses, such as an X-ray CT apparatus, in which a DRL value (sixth diagnostic reference level L6) is defined in accordance with an age group that is classified by the age range of children. In the example shown in Fig. 10, the DRL value (sixth diagnostic reference level L6) is defined as 30 mGy for children younger than 1 year, 40 mGy for children aged 1 year to younger than 5 years, 55 mGy for children aged 5 years to younger than 10 years, and 60 mGy for children aged 10 years to younger than 15 years. However, it is possible to set appropriate DRL values for ages by gradually changing the DRL values for children according to the ages rather than changing the DRL values in a stepwise manner for children according to the age groups.

Therefore, in this setting example, the controller 61 treats the DRL value (sixth diagnostic reference level L6) defined for each age group as a value for the median age of each group (where, a group of less than 1 year old is treated as a value for 1 year old). Then, the controller 61 calculates the DRL value (seventh diagnostic reference level L7) for each age by using the DRL value (sixth diagnostic reference level L6) set for the median age. For example, as the DRL value (seventh diagnostic reference level L7) for 2 years old, the controller 61 obtains the DRL value = 35 from the formula [30 + (40 - 30) × (2 - 1)/(3 - 1)] using the 30 mGy set for 1 year old and the 40 mGy set for 3 years old. As the DRL values (seventh diagnostic reference level L7) for 4 years old and 5 years old shown in Fig. 10, the controller 61 performs the same calculation as described above to obtain the respective DRL values = 43.3 and 46.7. The controller 61 obtains the DRL values (seventh diagnostic reference levels L7) by the same calculation for the ages in the group from 5 years old to less than 10 years old.

As described above, the controller 61 sets the seventh diagnostic reference levels L7 as the appropriate diagnostic reference levels for ages, using the DRL values (the sixth diagnostic reference levels L6) defined according to age groups.

In the example illustrated in Fig. 10, the controller 61 treats the DRL value (the sixth diagnostic reference level L6) defined for each age group as the value for the median age of each group. Not limited to this, the controller 61 may treat the DRL value (the sixth diagnostic reference level L6) defined for each age group as a value for the minimum or maximum age, and perform calculation similar to the above to obtain each DRL value (the seventh diagnostic reference level L7).

Further, in the example shown in Fig. 10, an example in which the target site is the head is shown, but the DRL values (the sixth diagnostic reference levels L6) corresponding to the age groups of the children are also defined for the chest, the abdomen, or the like. Therefore, even when the target site is the chest, the abdomen, or the like, the controller 61 may perform calculation similar to that described above and set an appropriate DRL value (seventh diagnostic reference level L7) for age.

With respect to the dynamic imaging performed by the radiographic imaging system 10 and the radiographic imaging control apparatus 20 illustrated in Fig. 1, it is assumed that the DRL value (sixth diagnostic reference level L6) is defined according to the age group of the child as illustrated in Fig. 10. Also in this case, the controller 61 may perform the same calculation as described above to set appropriate DRL values (seventh diagnostic reference levels L7) for ages.

As described above, in the present embodiment, the dose management apparatus 60 includes the second setting section 621. The second setting section 621 calculates the seventh diagnostic reference level L7 of the dose to the subject based on the sixth diagnostic reference level L6 of the dose defined for each age group and the age of the subject in a case where the target site of the subject is imaged by irradiation with radiation in the radiographic imaging apparatus. Then, the second setting section 621 sets the calculated seventh diagnostic reference level L7 as a value used for the dose management when the target site is imaged by the radiographic imaging apparatus.

Since the dose management apparatus 60 has the above-described configuration, it is possible to set an appropriate diagnostic reference level for radiation dose management of radiographic image capturing.

Next, the dose management apparatus 60 manages the dose information of the patient using the diagnostic reference levels set as described above.

For example, when the X-ray CT apparatus in the modality 70 captures a radiographic image, the controller of the X-ray CT apparatus calculates a dose index at the time of imaging based on the imaging execution conditions.

Then, the controller of the X-ray CT apparatus transmits the imaging execution conditions, a dose index, and the like to the image management apparatus 40, the dose management apparatus 60, and the like via the communication section in association with the captured radiographic image. Furthermore, in response to the operation, the dose management apparatus 60 may acquire the imaging execution conditions, dose index, and the like together with the radiographic image from the X-ray CT apparatus, the image management apparatus 40, or the like.

The controller 61 of the dose management apparatus 60 stores and accumulates the transmitted or acquired radiographic images, imaging execution conditions, dose indices, and the like in the storage section 62, and displays information for comparing the set diagnostic reference level with the dose index on the display section 24 for the patient who is the dose management target.

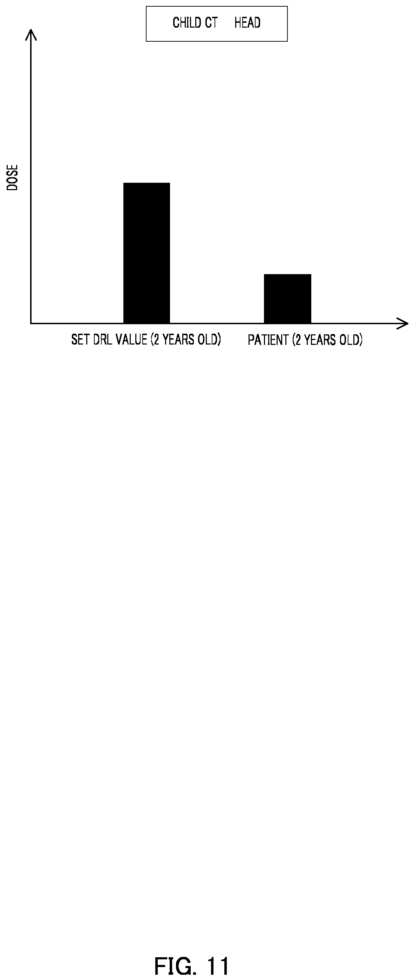

Here, Fig. 11 is a graph displayed for comparison in the dose management apparatus 60 shown in Fig. 9, and is a graph for comparing the DRL value shown in Fig. 10 with the exposure dose of the patient. In Fig. 11, since the patient is 2 years old, the DRL value of the pediatric CT of the head for 2 years old and the exposure dose of the patient of 2 years old are displayed and compared.

In this manner, the controller 61 provides information on dose management to the operator of the dose management apparatus 60 by displaying, on the display section 24, a graph for comparing the set diagnostic reference level with the exposure dose for a patient who is a dose management target. For example, the controller 61 may provide the operator of the dose management apparatus 60 with information for reviewing the currently set diagnostic reference level by displaying the exposure dose of a plurality of patients in a histogram for each examination item. In addition, based on the set diagnostic reference level and the exposure dose of the patient, the controller 61 may calculate the number of radiographic images (the number of frames) and imaging time that are allowable thereafter, and provide the calculated result to the radiographic imaging control apparatus 20 or the modality 70.

### (Setting Example 6)

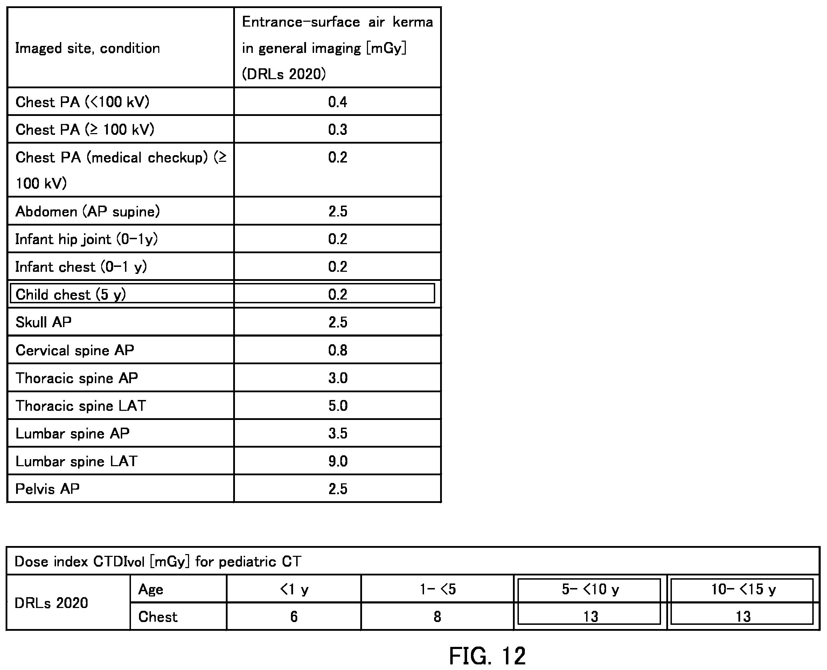

Fig. 12 is a diagram illustrating a DRL value (entrance-surface dose) in general imaging and a DRL value (dose indicator) set according to an age group of children in chest CT.

Also in general imaging, the DRL value is not defined for some examination items. For example, in the general imaging illustrated in Fig. 12, the child chest (5 years old) is defined, but the child chest (10 years old) is not defined.

Therefore, in this setting example, based on the DRL value (eighth diagnostic reference level L8) defined for the modality which is different from the target radiography apparatus, the controller 61 sets the DRL value (seventh diagnostic reference level L7) for the examination item which is not defined.

For example, in the example illustrated in Fig. 12, in a case where the DRL value (the seventh diagnostic reference level L7) for the child chest (10 years old) in the general imaging is set, the DRL value (the eighth diagnostic reference level L8) set according to the age group of children for the chest CT is referred to.

For CT, as illustrated in Fig. 12, the DRL value of 13 mGy is defined for ages 5 to younger than 10, and the DRL value of 13 mGy is defined for ages 10 to younger than 15. Based on these DRL values, the controller 61 treats the DRL value for the age of 5 as 13 mGy and the DRL value for the age of 10 as 13 mGy, and calculates the ratio of the latter DRL value to the former DRL value (13/13). In this example, the ratio is 1.

Next, the controller 61 multiplies the DRL value of 0.2 mGy specified for the child chest (5 years old) by the obtained ratio, to thereby set 0.2 (= 0.2 × 13/13) as the DRL value for the child chest (10 years old).

As described above, the controller 61 uses the DRL value (the eighth diagnostic reference level L8) defined for the modality different from the target radiographic imaging apparatus to set an appropriate DRL value (the seventh diagnostic reference level L7) for an undefined examination item.

Note that the example illustrated in Fig. 12 is an example. For example, for an undefined examination item other than those described above, an appropriate DRL value (the seventh diagnostic reference level L7) can be set using a DRL value (the eighth diagnostic reference level L8) defined for a modality different from the target radiographic image capturing apparatus.

For example, in dynamic imaging performed by the radiographic imaging system 10 and the radiographic imaging control apparatus 20 illustrated in Fig. 1, it is assumed that a DRL value for a child chest (5 years old) is defined, and a DRL value for a child chest (10 years old) is not defined. In this case, in the same manner as described above, an appropriate DRL value (seventh diagnostic reference level L7) can be set for the child chest (10 years old) using the DRL value (eighth diagnostic reference level L8) set according to the age group of the child in the chest CT.

### (Setting Example 7)

In the setting example 5 and the setting example 6, the influence of each site is not considered, but the diagnostic reference level may be set in consideration of the influence of each site.

For example, since the amount of radiation transmitted through the chest varies depending on the respiratory state, it is better to correct the diagnostic reference level according to the respiratory state. Specifically, since the amount of transmitted radiation is larger in imaging in the state of inhaling than in imaging in the state of exhaling, the diagnostic reference level can be set lower. For example, in X-ray imaging of the posteroanterior view of the chest in a medical checkup, imaging is performed in a state of inhaling, and therefore the diagnostic reference level can be set lower.

In the present setting example, the controller 61 includes the second correction section 622, and the second correction section 622 corrects the DRL value (seventh diagnostic reference level L7) using the correction factor α for imaging of the posteroanterior view of the chest in a state of inhaling. The correction factor α in this case is, for example, 0.9. In this case, since the DRL value (seventh diagnostic reference level L7) for the "child chest (5 years old)" shown in Fig. 12 is "0.2", when the posteroanterior view of the chest is imaged in a state of inhaling, this is multiplied by the correction factor α= 0.9, and "0.18" is set as the DRL value after the correction.

Furthermore, since the transmission amount of X-rays also changes depending on the body thickness, the DRL value (the seventh diagnostic reference level L7) may be corrected according to the body thickness. For example, since the lateral view of the lumbar spine has a thicker body thickness than other portions, the second correction section 622 of the controller 61 corrects the DRL value (seventh diagnostic reference level L7) using the correction factor α also for imaging of the lateral view of the lumbar spine. When the correction factor α in this case is, for example, 1.3, the DRL value (seventh diagnostic reference level L7) is "9.0" for the "Lumbar spine LAT" shown in Fig. 12, and "11.7" is thus set as the corrected DRL value by multiplying the DRL value by the correction factor α= 1.3.

As described above, the controller 61 corrects the seventh diagnostic reference level L7 serving as the diagnostic reference level in accordance with the site.

In the setting example 5 and the setting example 6, the seventh diagnostic reference level L7 of the dose to the subject is calculated on the basis of the DRL values defined for age groups grouped by age ranges, but an item other than the age may be used as long as the item is divided by a numerical range. The items may be, for example, numerical settings related to radiation irradiation, such as a tube voltage.

Note that the following supplementary notes will be further disclosed with respect to the above description.

### (Supplementary Note 1)

A dose management apparatus, including:
a second setting section that calculates a seventh reference value of a dose to a subject based on a sixth reference value of the dose defined for each group of items classified by a numerical range for a case where a target site of the subject is imaged by irradiation with radiation by a radiographic imaging apparatus and a numerical value of the subject for the item and sets the seventh reference value as a value used for dose management in a case where the target site is imaged by the radiographic image capturing apparatus.

### (Supplementary Note 2)

The dose management apparatus according to Supplementary Note 1, in which
the second setting section calculates the seventh reference value by using the sixth reference value of the group as a value corresponding to a median value, a minimum value, or a maximum value of the numerical range of the group.

### (Supplementary Note 3)

The dose management apparatus according to Supplementary Note 2, further including:
a second correction section that corrects the seventh reference value according to the target site.

### (Supplementary Note 4)

The dose management apparatus according to Supplementary Note 3, in which
the second correction section corrects the seventh reference value according to a body thickness of the target site.

### (Supplementary Note 5)

The dose management apparatus according to Supplementary Note 1, further including:
a second correction section that corrects the seventh reference value according to a respiratory state of the subject when the target site is a chest.

### (Supplementary Note 6)

The dose management apparatus according to Supplementary Note 1, in which:
the second setting section calculates the seventh reference value by using, instead of the sixth reference value, an eighth reference value of the dose defined for each of the groups when the target site is imaged by a radiographic imaging apparatus different from the radiographic imaging apparatus.

### (Supplementary Note 7)

The dose management apparatus according to Supplementary Note 1, in which:
the item is one of an age of the subject and a numerical value setting related to the irradiation of the radiation.

### (Supplementary Note 8)

A dose setting method performed by a dose management apparatus and including:
calculating a seventh reference value of a dose to a subject based on a sixth reference value of the dose defined for each group of items classified by a numerical range for a case where a target site of the subject is imaged by irradiation with radiation by a radiographic imaging apparatus and a numerical value of the subject for the item and setting the seventh reference value as a value used for dose management in a case where the target site is imaged by the radiographic image capturing apparatus.

### (Supplementary Note 9)

A dose setting program for causing a computer of a dose management apparatus to execute:
a process of calculating a seventh reference value of a dose to a subject based on a sixth reference value of the dose defined for each group of items classified by a numerical range for a case where a target site of the subject is imaged by irradiation with radiation by a radiographic imaging apparatus and a numerical value of the subject for the item and setting the seventh reference value as a value used for dose management in a case where the target site is imaged by the radiographic image capturing apparatus.

The above-described embodiments are merely examples for implementing the present invention, and the technical scope of the present invention should not be interpreted in a limited manner by these embodiments. That is, the present invention can be implemented in various forms without departing from the spirit or main features thereof.

Although embodiments of the present invention have been described and illustrated in detail, the disclosed embodiments are made for purpose of illustration and example only and not limitation. The scope of the present invention should be interpreted by terms of the appended claims.

## Claims

1. A dose management apparatus (60), comprising:
a ratio acquisition section (611) that acquires a ratio between a first reference value of a dose defined for a case where a first site of a subject is imaged as a still image by irradiation with radiation and a second reference value of a dose defined for a case where the first site is imaged as a dynamic image by irradiation with radiation; and
a first setting section (612) that sets, based on the ratio and a third reference value of a dose for a case where a second site is imaged as the still image, a fourth reference value of a dose for a case where the second site is imaged as the dynamic image.

2. The dose management apparatus (60) according to claim 1, wherein:
the second site is different from the first site, and
the third reference value is a value defined for a case where the second site is imaged as a still image.

3. The dose management apparatus (60) according to claim 1, wherein:
the second site is the same site as the first site, and
the third reference value is a value set for the case where the second site is imaged as the still image, and is a value different from the first reference value.

4. The dose management apparatus (60) according to claim 1, wherein
the ratio acquisition section (611) obtains the ratio using, instead of the first reference value, a fifth reference value of a dose uniquely defined for an imaging facility for the case where the first site is imaged as the still image.

5. The dose management apparatus (60) according to claim 1, further comprising:
a first correction section (613) that corrects the fourth reference value in accordance with the second site.

6. The dose management apparatus (60) according to claim 5, wherein
the first correction section (613) corrects the fourth reference value in accordance with a body thickness of the second site.

7. The dose management apparatus (60) according to claim 1, further comprising:
a first correction section (613) that corrects the fourth reference value in accordance with a respiratory state of the subject when the second site is a chest.

8. A dose setting method performed by a dose management apparatus (60), comprising:
obtaining a ratio between a first reference value of a dose defined for a case in which a first site of a subject is imaged as a still image by irradiation with radiation and a second reference value of a dose defined for a case in which the first site is imaged as a dynamic image by irradiation with radiation, and
setting, based on the ratio and a third reference value of a dose for a case where a second site is imaged as the still image, a fourth reference value of a dose for a case where the second site is imaged as the dynamic image.

9. A dose setting program for causing a computer of a dose management apparatus (60) to execute:
a process of obtaining a ratio between a first reference value of a dose defined for a case in which a first site of a subject is imaged as a still image by irradiation with radiation and a second reference value of a dose defined for a case in which the first site is imaged as a dynamic image by irradiation with radiation, and
a process of setting, based on the ratio and a third reference value of a dose for a case in which a second site is imaged as the still image, a fourth reference value of a dose for a case in which the second site is imaged as the dynamic image.
